# EUROPEAN PATENT APPLICATION

(11) **EP 3 176 130 A1**
(43) Date of publication of application: **07.06.2017**
(21) Application number: 15826471.3
(22) Date of filing: 17.07.2015
(51) Int. Cl.: C02F 1/50, B01F 3/04, B01F 5/04, B01F 15/02, B01J 19/08, H05H 1/24

(54) **TREATMENT DEVICE, STERILIZATION DEVICE, STERILIZATION WATER, AND STERILIZATION METHOD**

(30) Priority: 28.07.2014 US 201462029703 P
(71) Applicant: NGK Insulators, Ltd., Nagoya-shi, Aichi 467-8530 (JP)
(72) Inventor: SHIMIZU Hideki, Nagoya-shi Aichi 467-8530 (JP); IMANISHI Yuichiro, Nagoya-shi Aichi 467-8530 (JP); YAMADA Kazunari, Nagoya-shi Aichi 467-8530 (JP); YOKOYAMA Takashi, Nagoya-shi Aichi 467-8530 (JP)
(74) Representative: Naylor, Matthew John
(86) International application number: PCT/JP2015/070520
(87) International publication number: WO 2016/017456

(57) **Abstract**

The present invention relates to a treatment device, sterilization device, sterile water, and sterilization method. Treatment devices (10A, 10B) and a sterilization device (70) comprise a liquid reservoir part (14) having a liquid to be treated (12), a plasma generating part (16) for generating plasma on a liquid surface (12a) of the liquid to be treated (12), and a gas bubble feed part (20) for producing gas bubbles (18) encapsulating the generated plasma on the liquid surface (12a) and feeding the bubbles into the liquid to be treated (12). Moreover, plasma is generated on the liquid surface (12a) of the liquid to be treated (12) and the gas bubbles (18) encapsulating the generated plasma are generated and fed into the liquid to be treated (12).

## Description

### Technical Field

The present invention relates to a treatment apparatus (device), a sterilization apparatus (device), a sterilization water, and a sterilization method utilizing a plasma.

### Background Art

Liquid treatment apparatuses and sterilization methods utilizing plasma are described, e.g., in Japanese Patent Nos. 5099612 and 4408957.

A liquid treatment apparatus described in Japanese Patent No. 5099612 contains a gas discharge portion and a microbubble generation portion integrally connected thereto. The liquid treatment apparatus is immersed in a treatment subject liquid (a liquid to be treated) in a liquid tank. The gas discharge portion is connected to a gas supply portion for supplying a gas via a gas supply tube. The microbubble generation portion is connected to a circulation pump immersed in the liquid via a liquid supply tube.

In the gas discharge portion, a gas discharge is generated between both electrodes under atmospheric pressure, whereby a plasma is generated in the gas to prepare an activated gas. In the microbubble generation portion, the activated gas prepared in the gas discharge portion is converted to microbubbles having a diameter of 1 to 100 µm in the treatment subject liquid, and thus-obtained gas-liquid mixture fluid is supplied into the treatment subject liquid.

In a sterilization method described in Japanese Patent No. 4408957, radicals generated by a plasma are brought into contact with a liquid having a pH value of 4.8 or less in a sterilization process. A sterilization apparatus for killing bacteria present in or on the liquid is used in this method. The sterilization apparatus contains a pH control device for controlling the liquid pH at 4.8 or less and a plasma generation device for emitting the plasma toward the liquid.

### Summary of Invention

However, in the liquid treatment apparatus described in Japanese Patent No. 5099612, the gas discharge portion and the microbubble generation portion integrally connected to each other have to be immersed in the treatment subject liquid. It is necessary to increase the volume of the treatment subject liquid to sufficiently immerse the liquid treatment apparatus. Therefore, when a vessel for the treatment subject liquid (such as a reservoir tank) has a predetermined limited volume, the amount (concentration) of the microbubbles can be increased only to a limited extent, even if the number of the liquid treatment apparatuses to be immersed is increased. Thus, the concentration of the generated microbubbles cannot be satisfactorily increased. Consequently, for example, a large amount of radicals having a sterilization effect cannot be generated in the liquid in some cases.

In the method described in Japanese Patent No. 4408957, the plasma is emitted toward the liquid. In a case where the plasma is a gas hardly-soluble in the liquid, a large amount of radicals insoluble in water may be generated. In addition, in sterilization of a food etc., an organic substance such as food may be damaged by the liquid having a pH value of 4.8 or less (the acidic liquid).

In view of the above problems, an object of the present invention is to provide a treatment apparatus, which can supply a high concentration of microbubbles into a treatment subject liquid without immersing the apparatus in the treatment subject liquid, and which can reduce damage to a treatment target material (such as an organic substance such as food) even in the case of using an acidic liquid as the treatment subject liquid.

Another object of the present invention is to provide a sterilization apparatus, which utilizes the above treatment apparatus and thereby can produce a sterilization water containing a large amount of radicals having a sterilization effect.

A further object of the present invention is to provide a sterilization water containing a large amount of radicals having a sterilization effect.

A still further object of the present invention is to provide a sterilization method, which can efficiently sterilize a treatment target material by a sterilization water containing a large amount of radicals having a sterilization effect.
[1] According to a first aspect of the present invention, there is provided a treatment apparatus comprising a liquid reservoir portion configured to store a treatment subject liquid, a plasma generation portion configured to generate a plasma on or above a liquid surface of the treatment subject liquid, and a bubble supply portion configured to generate a bubble containing the generated plasma on or above the liquid surface and to supply the bubble into the treatment subject liquid.
   In the treatment apparatus, the plasma is generated on or above the liquid surface of the treatment subject liquid, the bubble containing the generated plasma is generated on or above the liquid surface, and the generated bubble is supplied into the treatment subject liquid. The term "on or above the liquid surface" means "at the same level as the liquid surface or above the liquid surface". Therefore, the bubble containing the plasma can be supplied into the treatment subject liquid without immersing the treatment apparatus per se in the treatment subject liquid. Consequently, the number of the treatment apparatuses is not limited by the volume of a vessel for the treatment subject liquid (such as a reservoir tank) and can be easily increased. Furthermore, the concentration of the bubble to be supplied into the liquid, i.e., the bubble amount (number) per unit volume, can be increased.
[2] In the first aspect of the present invention, the treatment subject liquid may be controlled to be an acidic liquid. In general, when the treatment subject liquid is controlled to be an acidic liquid and when a treatment target material is immersed in the acidic treatment subject liquid, the treatment target material is damaged by the acidic treatment subject liquid. In contrast, in the present invention, because the concentration of the bubble to be supplied into the treatment subject liquid can be increased, the damage to the treatment target material can be reduced. Furthermore, when the treatment subject liquid is controlled to be an acidic liquid and when the bubble is supplied into the acidic treatment subject liquid, the gas in the bubble can be dissolved more rapidly, and a plasma active species or a reaction product derived therefrom can be efficiently dissolved in the acidic treatment subject liquid. In addition, when the treatment subject liquid is controlled to be an acidic liquid, the bubble exhibits a lower resistance (contact resistance) to the acidic treatment subject liquid, so that the bubble can be readily transferred in the acidic treatment subject liquid. Consequently, for example, radicals having a sterilization effect can be widely spread in a short time, the concentration of the radicals can be increased in the treatment subject liquid, and a sterilization water having a high sterilization effect can be produced from the treatment subject liquid.
[3] In the first aspect of the present invention, the bubble supply portion may be configured to introduce the plasma from the plasma generation portion into a liquid to generate the bubble, wherein the liquid is supplied into the liquid reservoir portion.
   The above liquid is supplied into the liquid reservoir portion for storing the treatment subject liquid. The plasma is introduced into the liquid in the process of supplying the liquid into the liquid reservoir portion, whereby the bubble containing the plasma is generated in the liquid. The generated bubble is supplied, together with the liquid, into the treatment subject liquid. Consequently, the plasma can be generated on or above the liquid surface of the treatment subject liquid, the bubble containing the generated plasma can be generated on or above the liquid surface, and the generated bubble can be supplied into the treatment subject liquid.
[4] In this case, the bubble supply portion may contain an aspirator having a venturi portion on a flow path configured to supply the liquid into the liquid reservoir portion, and the bubble supply portion may be configured to introduce the plasma from the plasma generation portion through the venturi portion to generate the bubble.
   When the liquid flows through the venturi portion in the aspirator, the flow speed is increased to lower the pressure due to the venturi effect in the venturi portion. Therefore, the plasma generated in the plasma generation portion flows into the venturi portion having the lowered pressure, whereby the bubble containing the plasma is generated in the liquid. The liquid containing the bubble is supplied through the aspirator into the treatment subject liquid in the liquid reservoir portion. By appropriately selecting the diameter and the like of the venturi portion, a microbubble having a diameter of 50 µm or less can be generated as the bubble.
   In a case where the bubble is downsized to obtain the microbubble, the contact area between the liquid and a plasma active species or a reaction product derived therefrom can be increased. Furthermore, in this case, the inner pressure and the solubility can be increased due to the downsizing, whereby the plasma active species or the reaction product derived therefrom can be efficiently dissolved in the treatment subject liquid.
[5] In this case, the treatment apparatus may have a space formed on or above the liquid surface of the treatment subject liquid, a plasma generating gas may be introduced into the space, the plasma generation portion and the bubble supply portion may be placed in the space, and the plasma generation portion may be configured to generate the plasma based on introduction of the plasma generating gas into the space.
   The plasma generating gas is introduced into the space and then supplied into the plasma generation portion. The plasma generation portion is configured to generate the plasma based on the supply of the plasma generating gas, and the plasma is introduced into the liquid in the middle of supply into the liquid reservoir portion. The bubble containing the plasma can be generated in the liquid in this manner.
[6] In this case, the liquid may be the treatment subject liquid circulated and supplied from the liquid reservoir portion.
   When the treatment subject liquid, which contains the bubble containing the plasma, is circulated and supplied from the liquid reservoir portion and is used as the above-described liquid to be supplied into the liquid reservoir portion, the amount (number) of the bubble per unit volume can be increased, and the concentration (the amount per unit volume) of the plasma active species or the reaction product derived therefrom can be increased in the treatment subject liquid.
[7] In the first aspect of the present invention, the treatment apparatus may contain at least one combination of the one plasma generation portion and the one bubble supply portion.
   Most of the plasma generated in the one plasma generation portion can be introduced through the one bubble supply portion into the liquid, whereby a large number of the bubbles can be generated in the liquid.
[8] In the first aspect of the present invention, the treatment apparatus may contain at least one combination of the one plasma generation portion and a plurality of the bubble supply portions.
   The plasma generated in the one plasma generation portion can be introduced through the plurality of bubble supply portions into the liquid, whereby a large number of the bubbles can be dispersed and supplied into the treatment subject liquid.
[9] In this case, the plurality of bubble supply portions may be arranged on a circle centered at the one plasma generation portion.
   The plasma generated in the one plasma generation portion can be distributed approximately uniformly in the plurality of bubble supply portions arranged circularly. Thus, the one plasma generation portion can be used as a plasma source for the plurality of bubble supply portions. The number of the plasma sources can be reduced, and the structure of the treatment apparatus can be simplified. In addition, a large amount of the bubble can be efficiently dispersed in the treatment subject liquid, and for example, the radical concentration can be rapidly uniformized in the treatment subject liquid.
[10] According to a second aspect of the present invention, there is provided a sterilization apparatus comprising a liquid reservoir portion configured to store a treatment subject liquid, a plasma generation portion configured to generate a plasma on or above a liquid surface of the treatment subject liquid, and a bubble supply portion configured to generate a bubble containing the generated plasma on or above the liquid surface and to supply the bubble into the treatment subject liquid. A sterilization water containing a large amount of radicals having a sterilization effect can be produced by the sterilization apparatus.
[11] In the second aspect of the present invention, the treatment subject liquid may be controlled to be an acidic liquid.
[12] According to a third aspect of the present invention, there is provided a sterilization water produced by the above-described sterilization apparatus. The sterilization water contains a large amount of radicals having a sterilization effect.
[13] According to a fourth aspect of the present invention, there is provided a sterilization method comprising the steps of generating a plasma on or above a liquid surface of a treatment subject liquid, generating a bubble containing the generated plasma on or above the liquid surface, and supplying the bubble into the treatment subject liquid. In this method, a treatment target material can be efficiently sterilized by a sterilization water containing a large amount of radicals having a sterilization effect.
[14] In the fourth aspect of the present invention, the treatment subject liquid may be controlled to be an acidic liquid.

As described above, the treatment apparatus of the present invention can supply a high concentration of microbubbles into the treatment subject liquid without immersing the apparatus per se in the treatment subject liquid, and can reduce damage to the treatment target material (such as an organic substance in a food) even in the case of using the acidic treatment subject liquid.

The sterilization apparatus of the present invention can be used for producing the sterilization water containing a large amount of the radicals having the sterilization effect.

The sterilization water of the present invention, which contains a large amount of the radicals having the sterilization effect, can be used for efficiently sterilizing the treatment target material.

The sterilization method of the present invention can be used for efficiently sterilizing the treatment target material by the sterilization water containing a large amount of the radicals having the sterilization effect.

The above objects, features, and advantages of the present invention will become more apparent from the following description when taken in conjunction with the accompanying drawings.

### Brief Description of Drawings

FIG. 1 is a schematic structural view of a treatment apparatus according to a first embodiment (a first treatment apparatus);
FIG. 2 is a schematic structural view from above of the first treatment apparatus;
FIG. 3 is an explanatory view of a structure of a plasma generation portion and a bubble supply portion;
FIG. 4 is a front view of a structure of a first discharge electrode and a second discharge electrode in a discharge electrode portion;
FIG. 5 is a schematic structural view of a treatment apparatus according to a second embodiment (a second treatment apparatus); and
FIG. 6 is a schematic structural view from above of the second treatment apparatus.

### Description of Embodiments

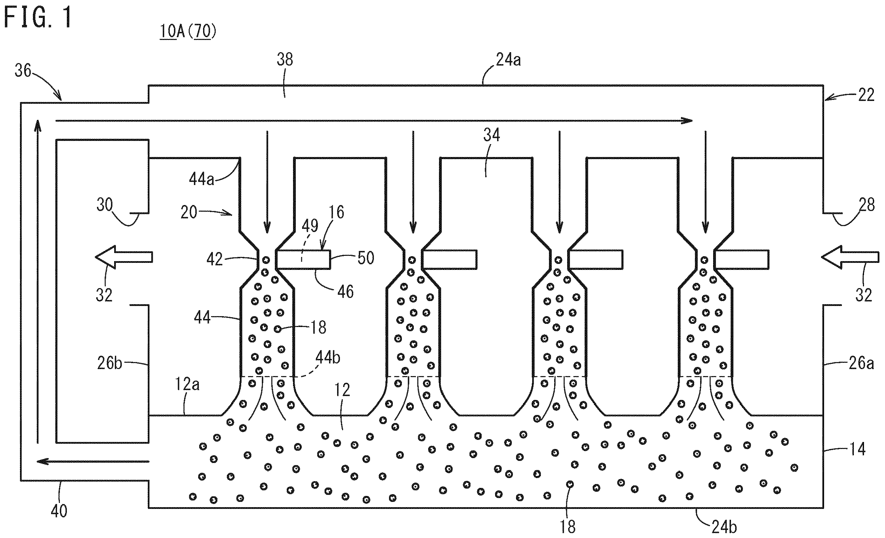

Several embodiments of the treatment apparatus, the sterilization apparatus, the sterilization water, and the sterilization method of the present invention will be described below with reference to FIGS. 1 to 6.

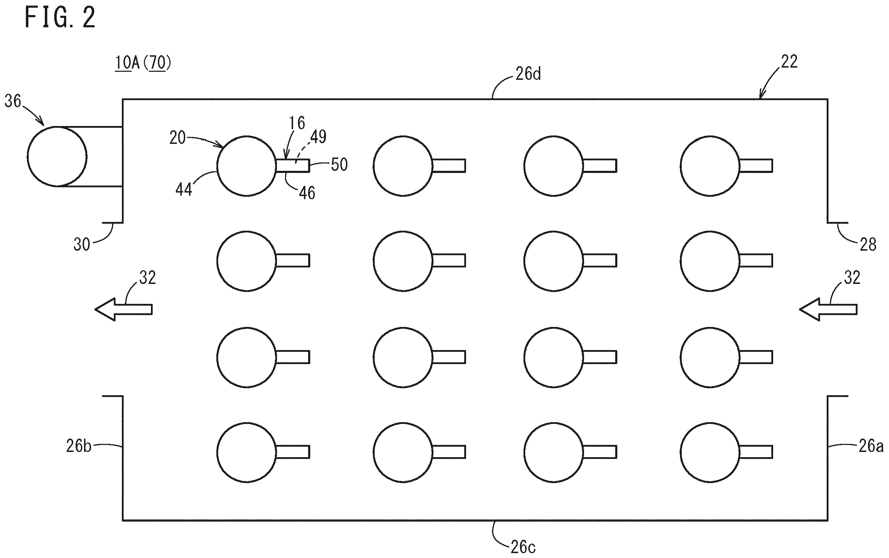

As shown in FIGS. 1 and 2, a treatment apparatus according to a first embodiment of the present invention (hereinafter referred to as the first treatment apparatus 10A) has a liquid reservoir portion 14 for storing a treatment subject liquid 12, plasma generation portions 16 for generating a plasma on or above a liquid surface 12a of the treatment subject liquid 12, and bubble supply portions 20 for generating bubbles 18 containing the generated plasma on or above the liquid surface 12a and for supplying the bubbles 18 into the treatment subject liquid 12. The treatment subject liquid 12 is an acidic liquid. For example, the pH value of the treatment subject liquid 12 is controlled at 4.8 or less. The term "on or above the liquid surface 12a" means "at the same level as the liquid surface 12a or above the liquid surface 12a".

More specifically, for example, the first treatment apparatus 10A has a rectangular housing 22. The housing 22 has an upper surface 24a, a lower surface 24b, and four side surfaces (a first side surface 26a to a fourth side surface 26d). In the housing 22, for example, a gas inlet 28 is formed on the first side surface 26a, and a gas outlet 30 is formed on a second side surface 26b opposite to the first side surface 26a. The liquid reservoir portion 14 is formed in a lower portion of the housing 22. In the housing 22, a space 34, into which a plasma generating gas 32 is introduced, is formed on or above the liquid surface 12a of the treatment subject liquid 12. The plasma generating gas 32 is supplied through the gas inlet 28 into the space 34 and is discharged through the gas outlet 30.

For example, on the second side surface 26b of the housing 22, a circulation flow path 36 for circulating the treatment subject liquid 12 extends from the liquid reservoir portion 14.

The circulation flow path 36 contains a liquid distribution portion 38 formed on an upper portion of the housing 22, and further contains a connection flow path 40 for connecting the liquid distribution portion 38 to the liquid reservoir portion 14. The connection flow path 40 extends outside of the second side surface 26b of the housing 22. For example, the treatment subject liquid 12 in the liquid reservoir portion 14 is drawn through the connection flow path 40 into the liquid distribution portion 38 by a pump (not shown). The pump may be a non-special common drawing pump.

The treatment subject liquid 12 in the liquid reservoir portion 14 is drawn by the pump through the connection flow path 40 into the upper liquid distribution portion 38. The drawn treatment subject liquid 12 is supplied into the liquid reservoir portion 14 again. Thus, the treatment subject liquid 12 is circulated through the circulation flow path 36 into the liquid reservoir portion 14. Incidentally, as viewed from the upper surface of the housing 22, the area of the liquid distribution portion 38 is approximately equal to that of the liquid reservoir portion 14.

Meanwhile, each of the bubble supply portions 20 extends from the liquid distribution portion 38 in the circulation flow path 36 toward the liquid reservoir portion 14. For example, the bubble supply portion 20 preferably contains an aspirator 44 having a venturi portion 42. An upper end 44a of the aspirator 44 is connected to the liquid distribution portion 38, and a lower end 44b is located on or above the liquid surface 12a of the treatment subject liquid 12. Thus, the aspirator 44 is hung down from the liquid distribution portion 38. The first treatment apparatus 10A contains a plurality of the aspirators 44. In the example of FIG. 2, four lines of the aspirators 44 are arranged from the first side surface 26a toward the second side surface 26b, four rows of the aspirators 44 are arranged from a third side surface 26c toward the fourth side surface 26d, and thus the first treatment apparatus 10A contains sixteen aspirators 44 in total.

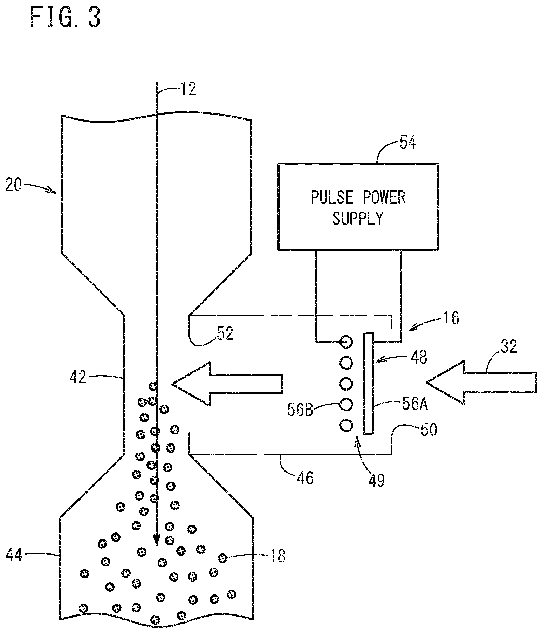

The plasma generation portion 16 is disposed on the venturi portion 42 in each of the aspirators 44. As shown in FIG. 3, each plasma generation portion 16 contains a casing 46, e.g., having a cylindrical shape, formed on the venturi portion 42, and further contains a discharge electrode portion 48 placed in the casing 46. In the casing 46, the area having the discharge electrode portion 48 is a plasma generation area 49. The casing 46 has a gas inlet 50 for introducing the plasma generating gas 32 and a through-hole 52 connected to the venturi portion 42. The inner space of the casing 46 is connected with the inner portion of the aspirator 44 by the through-hole 52. In the example of FIG. 2, the casings 46 (the gas inlets 50) in the plasma generation portions 16 are oriented in the same direction. The arrangement of the casings 46 is not limited thereto, and the casings 46 may be oriented in different directions.

A high-voltage pulse is applied from a pulse power supply 54 to the discharge electrode portion 48. The pulse power supply 54 may be attached to each of the plasma generation portions 16. Alternatively, one common pulse power supply 54 may be attached to a plurality of the plasma generation portions 16. In FIGS. 1 and 2, the pulse power supply 54 is not shown.

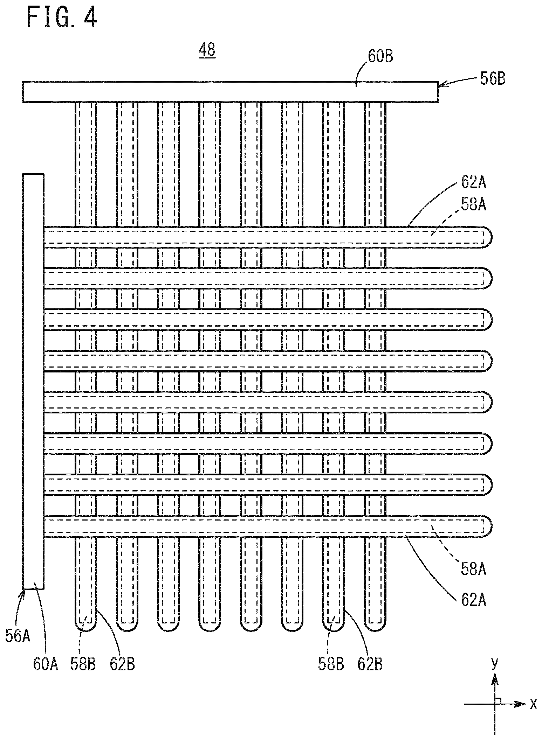

As shown in FIG. 4, the discharge electrode portion 48 contains a first discharge electrode 56A used as a positive electrode and a second discharge electrode 56B used as a negative electrode. The first discharge electrode 56A and the second discharge electrode 56B are arranged at a distance from each other along the flow direction. The fluid flows in the direction, in which the intensity of the electric field generated between the first discharge electrode 56A and the second discharge electrode 56B is maximized. Therefore, generation efficiency of the active species can be improved. The discharge electrode portion 48 may have the two-stage structure containing the first discharge electrode 56A and the second discharge electrode 56B, and may have a multi-stage structure such as a three-stage structure.

The first discharge electrode 56A contains a plurality of rod-shaped first conductors 58A, which extend in a first direction (an x direction) and are arranged in a second direction (a y direction) perpendicular to the first direction. The first discharge electrode 56A further contains a first common conductor 60A for connecting the plurality of first conductors 58A, and further contains first ceramic layers 62A applied to at least the first conductors 58A.

The second discharge electrode 56B contains a plurality of rod-shaped second conductors 58B, which extend in the second direction (the y direction) and are arranged in the first direction (the x direction). The second discharge electrode 56B further contains a second common conductor 60B for connecting the plurality of second conductors 58B, and further contains second ceramic layers 62B applied to at least the second conductors 58B.

The first conductor 58A and the second conductor 58B may contain copper, iron, tungsten, stainless steel, platinum, etc. The first ceramic layer 62A and the second ceramic layer 62B may contain alumina, silica, titania, zirconia, etc.

The operation of the first treatment apparatus 10A will be described below with reference to FIGS. 1 and 2. For example, the pump is driven to circulate the treatment subject liquid 12. Meanwhile, the pulse power supply 54 (see FIG. 3) is activated to generate the plasma in each plasma generation portions 16. Furthermore, the plasma generating gas 32 is supplied through the gas inlet 28 into the space 34 in the housing 22.

The treatment subject liquid 12 is drawn by the pump into the upper liquid distribution portion 38, transferred through the aspirators 44 in the bubble supply portions 20, and then returned to the liquid reservoir portion 14. This process is repeated to circulate the treatment subject liquid 12.

When the treatment subject liquid 12 flows through the venturi portion 42 in each aspirator 44, the flow speed is increased and the pressure is lowered due to the venturi effect in the venturi portion 42. Therefore, the plasma generating gas 32 supplied into the space 34 is drawn into each plasma generation portion 16, and introduced through the casing 46 into the venturi portion 42. The plasma is generated in the plasma generation portion 16, and introduced together with the plasma generating gas 32 into the low-pressure venturi portion 42. Consequently, the bubbles 18 containing the plasma are generated in the treatment subject liquid 12 in the venturi portion 42. The treatment subject liquid 12 containing the bubbles 18 is returned through the aspirator 44 to the treatment subject liquid 12 in the liquid reservoir portion 14.

The plasma is generated on or above the liquid surface 12a of the treatment subject liquid 12, the bubbles 18 containing the generated plasma are generated on or above the liquid surface 12a, and the generated bubbles 18 are supplied into the treatment subject liquid 12. Therefore, the bubbles 18 containing the plasma can be supplied into the treatment subject liquid 12 without immersing the first treatment apparatus 10A per se in the treatment subject liquid 12. Consequently, the number of the first treatment apparatuses 10A is not limited by the volume of the vessel for the treatment subject liquid 12 (in this case, the liquid reservoir portion 14) and can be easily increased. Furthermore, the concentration of the bubbles 18 to be supplied into the treatment subject liquid 12, i.e., the bubble amount (number) per unit volume, can be increased.

In general, when the treatment subject liquid 12 is controlled to be acidic and when a treatment target material is immersed in the acidic treatment subject liquid 12, the treatment target material is damaged by the acidic treatment subject liquid 12. In contrast, in this embodiment, because the concentration of the bubbles 18 which is supplied into the treatment subject liquid 12 can be increased, the damage to the treatment target material can be reduced. Furthermore, when the treatment subject liquid 12 is controlled to be acidic and when the bubbles 18 are supplied into the acidic treatment subject liquid 12, the gas in the bubbles 18 can be dissolved more rapidly, and a plasma active species or a reaction product derived therefrom can be efficiently dissolved in the acidic treatment subject liquid 12. In addition, when the treatment subject liquid 12 is controlled to be acidic, the bubbles 18 exhibit a lower resistance (contact resistance) to the acidic treatment subject liquid 12, so that the bubbles 18 can be readily transferred in the acidic treatment subject liquid 12. Consequently, the plasma active species or the reaction product derived therefrom can be widely spread in a short time, the concentration of the plasma active species or the reaction product derived therefrom can be increased in the treatment subject liquid 12.

The treatment subject liquid 12 which is supplied into the liquid reservoir portion 14 is used as the treatment subject liquid 12 circulated from the liquid reservoir portion 14, i.e., the treatment subject liquid 12 supplied with the bubbles 18 containing the plasma. Therefore, the amount (number) of the bubbles 18 per unit volume can be increased, and the concentration (amount per unit volume) of the plasma active species or the reaction product derived therefrom can be increased in the treatment subject liquid 12.

By appropriately selecting the diameter and the like of the venturi portion 42, a microbubble having a diameter of 50 µm or less can be generated as the bubble 18.

In the case of using the microbubble, the contact area between the treatment subject liquid 12 and the plasma active species or the reaction product derived therefrom can be increased. Furthermore, the inner pressure and the solubility can be increased due to the downsizing of the bubble 18, whereby the plasma active species or the reaction product derived therefrom can be efficiently dissolved in the treatment subject liquid 12.

In the first treatment apparatus 10A, most of the plasma generated in the one plasma generation portion 16 can be introduced through the one bubble supply portion 20 into the treatment subject liquid 12, whereby a large number of the bubbles 18 can be generated in the treatment subject liquid 12.

An oxygen-containing gas, a nitrogen-containing gas, a mixture gas of oxygen and nitrogen, or the like may be used as the plasma generating gas 32. In this case, radicals having a high sterilization effect can be generated by the plasma. Therefore, the radicals having the sterilization effect can be widely spread in the treatment subject liquid 12 in a short time, the concentration of the radicals can be increased in the treatment subject liquid 12, and for example a sterilization water having a high sterilization effect can be produced from the treatment subject liquid 12. Thus, the first treatment apparatus 10A can be used as a sterilization apparatus 70 for producing the sterilization water having the high sterilization effect.

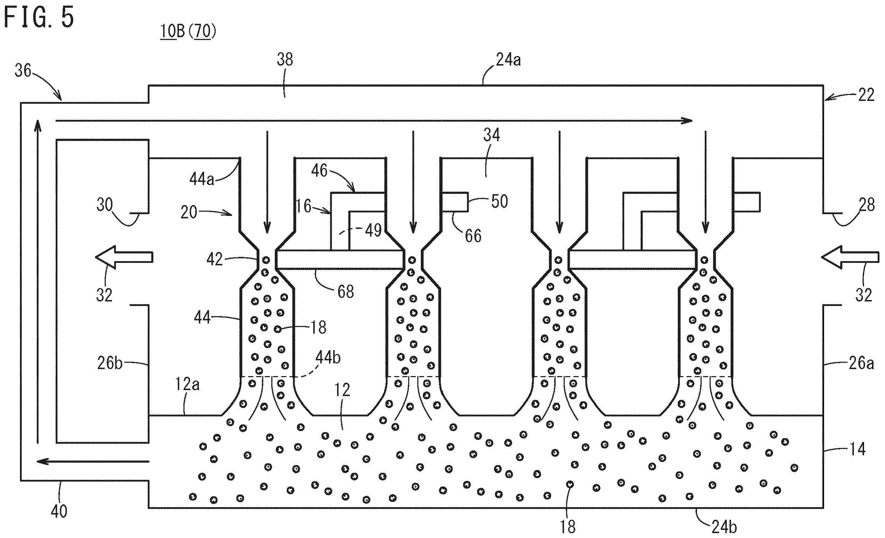

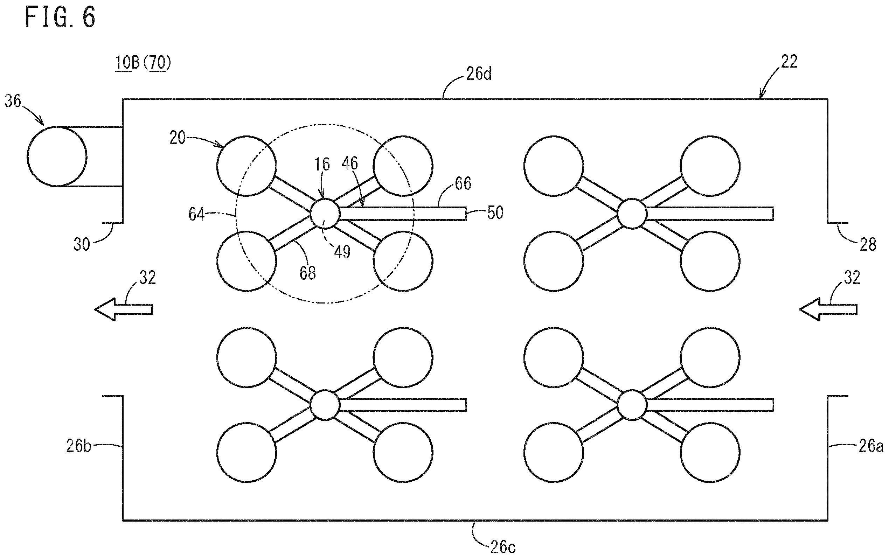

A treatment apparatus according to a second embodiment (hereinafter referred to as a second treatment apparatus 10B) will be described below with reference to FIGS. 5 and 6.

As shown in FIGS. 5 and 6, the second treatment apparatus 10B has approximately the same structure as the first treatment apparatus 10A, and is different in that it contains at least one combination of one plasma generation portion 16 and a plurality of the bubble supply portions 20.

Specifically, a plurality of the bubble supply portions 20 are arranged on a circle centered at the one plasma generation portion 16 (or arranged circularly around the one plasma generation portion 16). In the example of FIG. 6, four bubble supply portions 20 are arranged on a circle 64 (shown by a two-dot chain line) centered at the one plasma generation portion 16. As shown in FIGS. 5 and 6, the casing 46 has the gas inlet 50, a gas guide portion 66 for introducing the plasma generating gas 32 into the plasma generation area 49, and a branched portion 68 for introducing the gas containing the plasma from the plasma generation area 49 into the venturi portions 42 in the four bubble supply portions 20.

The second treatment apparatus 10B has the same advantageous effects as the first treatment apparatus 10A.

In particular, in the second treatment apparatus 10B, the plasma generated in the one plasma generation portion 16 can be introduced through the plurality of bubble supply portions 20 into the treatment subject liquid 12, whereby a large number of the bubbles 18 can be dispersed and supplied into the treatment subject liquid 12.

The plasma generated in the one plasma generation portion 16 can be distributed approximately uniformly in the plurality of the bubble supply portions 20 arranged circularly. Thus, the one plasma generation portion 16 can be used with the plasma generation area 49 for the plurality of bubble supply portions 20. The number of the plasma generation areas 49 can be reduced, and the structure of the second treatment apparatus 10B can be simplified. In addition, a large amount of the bubbles 18 can be efficiently dispersed in the treatment subject liquid 12, and for example, the radical concentration can be rapidly uniformized in the treatment subject liquid 12.

The treatment apparatus, the sterilization apparatus, the sterilization water, and the sterilization method of the present invention are not particularly limited to the aforementioned embodiments. Various changes and modifications may be made to the embodiments without departing from the scope of the invention.

## Claims

1. A treatment apparatus comprising:
a liquid reservoir portion (14) configured to store a treatment subject liquid (12),
a plasma generation portion (16) configured to generate a plasma on or above a liquid surface (12a) of the treatment subject liquid (12), and
a bubble supply portion (20) configured to generate a bubble (18) containing the generated plasma on or above the liquid surface (12a) and to supply the bubble (18) into the treatment subject liquid (12).

2. The treatment apparatus according to claim 1, wherein the treatment subject liquid (12) is controlled to be an acidic liquid.

3. The treatment apparatus according to claim 1 or 2, wherein the bubble supply portion (20) is configured to introduce the plasma from the plasma generation portion (16) into a liquid to generate the bubble (18), the liquid being supplied into the liquid reservoir portion (14).

4. The treatment apparatus according to claim 3, wherein:
the bubble supply portion (20) contains an aspirator (44) having a venturi portion (42) on a flow path configured to supply the liquid into the liquid reservoir portion (14), and
the bubble supply portion (20) is configured to introduce the plasma from the plasma generation portion (16) through the venturi portion (42) to generate the bubble (18).

5. The treatment apparatus according to claim 3 or 4, wherein:
the treatment apparatus has a space (34) formed on or above the liquid surface (12a) of the treatment subject liquid (12),
a plasma generating gas (32) is introduced into the space (34),
the plasma generation portion (16) and the bubble supply portion (20) are placed in the space (34), and
the plasma generation portion (16) is configured to generate the plasma based on introduction of the plasma generating gas (32) into the space (34).

6. The treatment apparatus according to any one of claims 3 to 5, wherein the liquid is the treatment subject liquid (12) circulated and supplied from the liquid reservoir portion (14).

7. The treatment apparatus according to any one of claims 1 to 6, wherein the treatment apparatus contains at least one combination of the one plasma generation portion (16) and the one bubble supply portion (20).

8. The treatment apparatus according to any one of claims 1 to 6, wherein the treatment apparatus contains at least one combination of the one plasma generation portion (16) and a plurality of the bubble supply portions (20).

9. The treatment apparatus according to claim 8, wherein the plurality of bubble supply portions (20) are arranged on a circle centered at the one plasma generation portion (16) or arranged circularly around the one plasma generation portion (16).

10. A sterilization apparatus comprising:
a liquid reservoir portion (14) configured to store a treatment subject liquid (12),
a plasma generation portion (16) configured to generate a plasma on or above a liquid surface (12a) of the treatment subject liquid (12), and
a bubble supply portion (20) configured to generate a bubble (18) containing the generated plasma on or above the liquid surface (12a) and to supply the bubble (18) into the treatment subject liquid (12).

11. The sterilization apparatus according to claim 10, wherein the treatment subject liquid (12) is controlled to be an acidic liquid.

12. A sterilization water produced by the sterilization apparatus according to claim 10 or 11.

13. A sterilization method comprising the steps of:
generating a plasma on or above a liquid surface (12a) of a treatment subject liquid (12),
generating a bubble (18) containing the generated plasma on or above the liquid surface (12a), and
supplying the bubble (18) into the treatment subject liquid (12).

14. The sterilization method according to claim 13, wherein the treatment subject liquid (12) is controlled to be an acidic liquid.
